# EUROPEAN PATENT APPLICATION

(11) **EP 3 695 825 A1**
(43) Date of publication of application: **19.08.2020**
(21) Application number: 20156667.6
(22) Date of filing: 11.02.2020
(51) Int. Cl.: A61K 8/06, A61K 8/49, A61K 8/92, A61Q 5/00, A61Q 5/02

(54) **HAIR CARE COMPOSITION**

(30) Priority: 15.02.2019 WO PCT/CN2019/075255; 04.04.2019 EP 19167422
(71) Applicant: Unilever PLC, London Greater London EC4Y 0DY (GB); Unilever N.V., 3013 AL Rotterdam (NL)
(72) Inventor: COLLINS, Luisa, Zoe, Bebington, Wirral, Merseyside CH63 3JW (GB); CHEN, Guoqiang, Shanghai, 200335 (CN); PU, Mingming, Shanghai, 200335 (CN)
(74) Representative: Tansley, Sally Elizabeth

(57) **Abstract**

Cosmetic use of a combination of piroctone olamine and coconut oil in a shampoo to decrease the level of genus Malassezia species on the human scalp.

## Description

### FIELD OF THE INVENTION

This invention is concerned with hair care compositions. More particularly, the present invention is concerned with the use of antidandruff hair care compositions.

### Background of the Invention

Anti-dandruff benefit has also been provided through hair care compositions. Dandruff is an issue that affects many people globally. The condition is manifested by the shedding of clumps of dead skin cells from the scalp. These are white in colour, and provide an aesthetically displeasing appearance. A factor that contributes to dandruff is certain members of the Malassezia yeasts. To combat these, anti-dandruff products have included certain a range of anti-dandruff agents which have anti-fungal activity, for example, zinc pyrithione, piroctone olamine (Octopirox®), azole based anti-fungal agents (e.g. climbazole, ketoconazole), selenium sulfide or combinations thereof.

Piroctone olamine has been used in treatment of dandruff because of its effectiveness against fungal infections. Dandruff is a common skin disorder manifesting by the shedding of clumps of dead skin cells which are yellow or white, giving an aesthetically displeasing appearance during daily life. Therefore, dandruff refers to the generation of skin surface flake or the flake itself. The finding that dandruff is fundamentally a Malassezia-based disorder remains prevalent. Consequently, anti-fungal actives such as piroctone olamine aiming to control yeast of the genus Malassezia remain as conventional antidandruff agents.

The need to lead a sustainable life style to use less water when showering or bathing is becoming increasingly important. This means there is a need for products that act quickly, necessitating less time when showering and thus result in less water usage. In addition, products that act quickly are seen as advantageous as they fit in with the consumer's busy life style.

Spec-Chem Ind: "Piroctone Olamine", potentially published 23 September 2009 (XP055625058) is a technical data sheet, which teaches that the agent responsible for the production of dandruff is Pityrosporum Ovale. It also teaches that piroctone olamine is active against Pilyrosporum Ovale.

WO-A-2013-093823 describes anti-fungal compositions comprising a broadly-described anti-fungal agent and an oil. The fungal agent can preferably be piroctone olamine. However, the composition is devoid of C11 or greater fatty acids and their esters, since these fatty acids/esters are said to serve as nutrients for the growth of fungus.

### Objects of the Invention

It is an objective of the present invention to provide the rapid decrease of the level of Malassezia species on a surface.

### Detailed Description of the Invention

In an embodiment, the invention provides the cosmetic use of piroctone olamine to decrease the level of Malassezia species on a surface.

In a further embodiment, there may be provided the use of piroctone olamine in decreasing the level of Malassezia species on a surface.

In both of the above embodiments, the decrease in the level of Malassezia species can be rapid, i.e., the invention can provide the cosmetic use of piroctone olamine to rapidly decrease the level of Malassezia species on a surface. "Rapid" in the context may mean less than 4 hours after application, preferably less than 2 hours. Conveniently "rapid" may mean less than 15 minutes, conveniently less than 10 minutes.

In all of the above embodiments, the use preferably includes the use of coconut oil with the piroctone olamine.

Herein, 'cosmetic use' should be understood to mean non-therapeutic use.

Preferably the surface is the skin. The skin according to the present invention is preferably human scalp.

These and other aspects, features and advantages will become apparent to those of ordinary skill in the art from a reading of the following detailed description and the appended claims. For the avoidance of doubt, any feature of one aspect of the present invention may be utilised in any other aspect of the invention. Any feature described as 'preferred' should be understood to be particularly preferred in combination with a further preferred feature or features. Herein, any feature of a particular embodiment may be utilized in any other embodiment of the invention. The word 'comprising' is intended to mean 'including' but not necessarily 'consisting of' or 'composed of'. In other words, the listed steps or options need not be exhaustive. The examples given in the description below are intended to clarify the invention but not to limit the invention. All percentages are weight/weight percentages unless otherwise indicated. Except in the operating and comparative examples, or where otherwise explicitly indicated, all numbers in this description indicating amounts of material or conditions of reaction, physical properties or materials and/or use are to be understood as modified by the word 'about'. Numerical ranges expressed in the format 'from x to y' are understood to include x and y, unless specified otherwise. When for a specific feature multiple preferred ranges are described in the format 'from x to y', it is understood that all ranges combining the different endpoints are also contemplated.

### DETAILED DESCRIPTION OF THE INVENTION

Piroctone olamine according to the present invention can be applied from a cosmetic composition, which is typically a transparent or opaque formulation such as emulsion, lotion, cream, paste or gel. Preferably, the cosmetic composition is a scalp treatment composition, which may be a rinse-off composition or a leave-on composition. A rinse-off composition is intended to be rinsed off the scalp of the user with water after use. Rinse-off compositions are preferred. A leave-on composition is intended not to be rinsed off the scalp of the user immediately after use (i.e. within at least the first 2 hours, preferably at least 4 hours after application of the composition). In the context of the present invention, rinse-off compositions include shampoos and conditioners, as well as treatment compositions which can be left on scalp for from 0.5 minute to up to 15 minutes, preferably from 1 minute to 10 minutes, more preferably 1 minutes to 10 minutes, prior to being rinsed off.

The preferred use of the cosmetic composition is in a shampoo, particularly a rinse-off shampoo. Shampoo compositions for use in the invention are generally aqueous, i.e. they have water or an aqueous solution, or a lyotropic liquid crystalline phase as their major component.

Suitably, the shampoo composition will comprise from 50 to 98%, preferably from 60 to 92% by weight water based on the total weight of the composition.

### Piroctone Olamine

Piroctone olamine is an olamine salt of the hydroxamic acid derivative piroctone. It is commonly known as piroctone ethanolamine with the trade name Octopirox®.

The piroctone olamine according to the present invention is a 1:1 compound of 1-hydroxy-4-methyl-6-(2,4,4-trimethylpentyl)-2(1*H*)-pyridinone with 2-aminoethanol and is also designated 1-hydroxy-4-methyl-6-(2,4,4-trimethylpentyl)-2(1*H*) pyridinone monoethanolamine salt. The CAS number is 68890-66-4 and the compound has the general formula (I) as below:

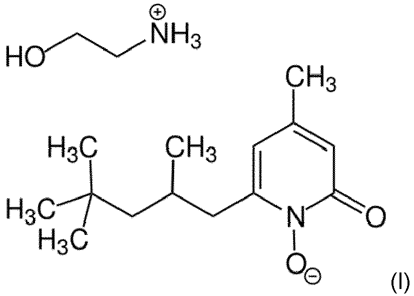

Piroctone olamine may be comprised in the cosmetic composition from 0.01 to 6.0 %, by weight of the total composition, preferably from 0.05 to 5 wt%, more preferably from 0.1 to 4 wt%.

### Anionic cleansing surfactant

The cosmetic composition may comprise one or more cleansing surfactants. Surfactants are compounds which have hydrophilic and hydrophobic portions that act to reduce the surface tension of the aqueous solutions they are dissolved in. Shampoo compositions according to the invention will generally comprise one or more cleansing surfactants, which are cosmetically acceptable and suitable for topical application to the hair. The cleansing surfactant may be chosen from anionic, non-ionic, amphoteric and zwitterionic compounds and mixtures thereof.

The total amount of cleansing surfactant in a shampoo composition for use in the invention is generally from 1 to 50%, preferably from 2 to 40%, more preferably from 4 to 25% by total weight surfactant based on the total weight of the composition.

Non-limiting examples cleansing surfactants include anionic cleansing surfactants including; alkyl sulphates, alkyl ether sulphates, alkaryl sulphonates, N-alkyl sarcosinates, alkyl phosphates, alkyl ether phosphates, acyl amino acid-based surfactants, alkyl ether carboxylic acids, acyl taurates, acyl glutamates, alkyl glycinates and salts thereof, especially their sodium, magnesium, ammonium and mono-, di- and triethanolamine salts. The alkyl and acyl groups in the preceding list generally contain from 8 to 18, preferably from 10 to 16 carbon atoms and may be unsaturated. The alkyl ether sulphates, alkyl ether phosphates and alkyl ether carboxylic acids and salts thereof may contain from 1 to 20 ethylene oxide or propylene oxide units per molecule.

Further non-limiting examples of cleansing surfactants may include non-ionic cleansing surfactants including; aliphatic (C₈ - C₁₈) primary or secondary linear or branched chain alcohols with alkylene oxides, usually ethylene oxide and generally having from 6 to 30 ethylene oxide groups. Other representative cleansing surfactants include mono- or di-alkyl alkanolamides (examples include coco mono-ethanolamide and coco monoisopropanolamide) and alkyl polyglycosides (APGs). Suitable alkyl polyglycosides for use in the invention are commercially available and include for example those materials identified as: Plantapon 1200 and Plantapon 2000 ex BASF. Other sugar-derived surfactants, which can be included in compositions for use in the invention include the C₁₀-C₁₈ N-alkyl (C₁-C₆) polyhydroxy fatty acid amides, such as the C₁₂-C₁₈ N-methyl glucamides, as described for example in WO 92 06154 and US 5194 639, and the N-alkoxy polyhydroxy fatty acid amides, such as C₁₀-C₁₈ N-(3-methoxypropyl) glucamide.

Additional non-limiting examples of cleansing surfactants include amphoteric or zwitterionic cleansing surfactants including; alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms.

Typical cleansing surfactants for use in shampoo compositions for use in the invention include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium cocoyl isethionate, sodium lauryl isethionate, lauryl ether carboxylic acid and sodium N-lauryl sarcosinate, sodium pareth sulphate, cocodimethyl sulphopropyl betaine, lauryl betaine, coco betaine, cocamidopropyl betaine, sodium cocoamphoacetate.

Preferred cleansing surfactants are sodium lauryl sulphate, sodium lauryl ether sulphate, sodium lauryl ether sulphosuccinate, ammonium lauryl sulphate, ammonium lauryl ether sulphate, sodium cocoyl isethionate and lauryl ether carboxylic acid, coco betaine, cocamidopropyl betaine, sodium cocoamphoacetate.

Mixtures of any of the foregoing anionic, non-ionic and amphoteric cleansing surfactants may also be suitable, preferably where the primary to secondary surfactant ratio is between 1:1 - 10:1, more preferably 2:1 - 9:1 and most preferably 3:1 - 8:1, based on the inclusion weight of the cleansing surfactant in the shampoo composition.

### Cationic deposition polymer

The cosmetic composition may comprise at least one cationic deposition polymer. Suitable cationic polymers may be homopolymers which are cationically substituted or may be formed from two or more types of monomers. The weight average (M_{w}) molecular weight of the polymers will generally be between 100 000 and 2 million daltons. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.
Amine substituted vinyl monomers and amines can be polymerised in the amine form and then converted to ammonium by quaternization.

The cationic polymers can comprise mixtures of monomer units derived from amine- and/or quaternary ammonium-substituted monomer and/or compatible spacer monomers.

Suitable (non-limiting examples of) cationic polymers include:
- cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and co-polymers of unsaturated carboxylic acids having from 3 to 5 carbon atoms, (as described in U.S. Patent 4,009,256);
- cationic polyacrylamides(as described in WO95/22311).

Other cationic polymers that can be used include cationic polysaccharide polymers, such as cationic cellulose derivatives, cationic starch derivatives, and cationic guar gum derivatives.

Cationic polysaccharide polymers suitable for use in compositions for use in the invention include monomers of the formula:

A-O-[R-N⁺(R¹)(R²)(R³)X⁻]

wherein: A is an anhydroglucose residual group, such as a starch or cellulose anhydroglucose residual. R is an alkylene, oxyalkylene, polyoxyalkylene, or hydroxyalkylene group, or combination thereof. R¹, R² and R³ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R¹, R² and R³) is preferably about 20 or less, and X is an anionic counterion.

Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation, for instance under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581). Examples of such materials include the polymer LR and JR series from Dow, generally referred to in the industry (CTFA) as Polyquaternium 10.

A particularly suitable type of cationic polysaccharide polymer that can be used is a cationic guar gum derivative, such as guar hydroxypropyltrimethylammonium chloride (commercially available from Rhodia in their JAGUAR trademark series). Examples of such materials are JAGUAR C13S, JAGUAR C14 and JAGUAR C17.

Mixtures of any of the above cationic polymers may be used.

Cationic polymer will generally be present in a shampoo composition for use in the invention at levels of from 0.01 to 5%, preferably from 0.02 to 1%, more preferably from 0.05 to 0.8% by total weight of cationic polymer based on the total weight of the composition.

### Other optional components

Preferably an aqueous shampoo composition for use in the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and crystalline long chain acyl derivative.

Suspending agent will generally be present in a shampoo composition for use in the invention at levels of from 0.1 to 10%, preferably from 0.15 to 6%, more preferably from 0.2 to 4% by total weight of suspending agent based on the total weight of the composition.

The cosmetic composition may optionally comprise one or more components, provided that the optional components are physically and chemically compatible with the essential components described hereinbefore, and do not otherwise unduly impair sensory, formulation rheology and conditioning performance. Individual concentrations of such optional components may range from 0.001% to 10% by weight of the total composition, preferably from 0.01% to 5%wt%. Such components may include conditioning agents, fragrance, dyes, pigments, pH adjusting agents, pearlescers or opacifiers, viscosity modifiers, preservatives, and natural hair nutrients such as botanicals, fruit extracts, sugar derivatives and amino acids.

One of the preferred optional components is a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich. Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and crystalline long chain acyl derivative.

A most preferred example is a crosslinked polyacrylate polymer.

Suspending agent, if included, will generally be present in the composition at a level of from 0.01 to 5 wt.%, preferably from 0.1 to 2.5 wt.%, more preferably from 0.25 to 1 wt.%.

Another preferred optional component is a conditioning agent, providing conditioning benefit. Typically, the most popular conditioning agents used in cosmetic compositions are water-insoluble oily materials such as mineral oils, naturally occurring oils such as triglycerides and silicone oils. Conditioning benefit is achieved by the oily material being deposited onto the skin resulting in the formation of a film, which makes the skin more lubricant and less dry. Preferably, the conditioning agent is non-volatile, meaning that it has a vapour pressure of less than 1000 Pa at 25°C.

Preferably, the composition comprises discrete dispersed droplets of a water-insoluble conditioning agent, which has a mean droplet diameter (D_{3,2}) of less than 15 microns, preferably less than 10 microns, more preferably less than 5 microns, most preferably less than 3 microns. The mean droplet diameter (D_{3,2}) of a water-insoluble conditioning agent may be measured by means of a laser light scattering technique, for example using a 2600D Particle Sizer from Malvern Instruments.

The water-insoluble conditioning agent may include non-silicone conditioning agent comprising non-silicone oily or fatty materials such as hydrocarbon oils, fatty esters and mixtures thereof. Preferably, the water-insoluble conditioning agent is emulsified silicone oil.

Suitable silicones include polydiorganosiloxanes, in particular polydimethylsiloxanes which have the CTFA designation dimethicone. Also suitable for use in compositions of this invention (particularly shampoos and conditioners) are polydimethyl siloxanes having hydroxyl end groups, which have the CTFA designation dimethiconol. Also suitable for use in compositions of this invention are silicone gums having a slight degree of cross-linking, as are described for example in WO 96/31188. Preferably, the silicone oil comprises dimethicone, dimethiconol or a mixture thereof.

The viscosity of the emulsified silicone itself (not the emulsion or the final hair care composition) is typically at least 10,000 cSt (centi-Stokes = mm²·S⁻¹) at 25°C, preferably at least 60,000 cSt, most preferably at least 500,000 cSt, ideally at least 1,000,000 cSt. Preferably the viscosity does not exceed 10⁹ cSt for ease of formulation. Suitable methods for measuring the kinematic viscosity of silicone oils are known to those skilled in the art, e.g. capillary viscometers. For high viscosity silicones, a constant stress rheometer can be used to measure viscosity.

Suitable emulsified silicones for use in the compositions are available as pre-formed silicone emulsions from suppliers of silicones such as Dow Corning and GE silicones. The use of such pre-formed silicone emulsion is preferred for ease of processing and control of silicone particle size. Such pre-formed silicone emulsions will typically additionally comprise a suitable emulsifier and may be prepared by a chemical emulsification process such as emulsion polymerisation, or by mechanical emulsification using a high shear mixer.

Examples of suitable pre-formed silicone emulsions include DC1785, DC1788, DC7128, all available from Dow Corning. These are emulsions of dimethiconol/dimethicone.

Another class of silicones which may be used are functionalized silicones such as amino functional silicones, meaning a silicone containing at least one primary, secondary or tertiary amine group, or a quaternary ammonium group. Examples of suitable amino functional silicones include polysiloxanes having the CTFA designation "amodimethicone."

Preferably, silicone emulsion droplets are blended with certain types of surface active block polymers of a high molecular weight to form silicone emulsions, as described for example in WO03/094874. One preferred form of the surface active block polymer having polyoxypropylene and polyoxyethylene groups as the hydrophobic and hydrophilic part respectively has Formula V and has the CTFA designation poloxamer, known commercially under the trade name "Pluronic" from BASF:

V) HO(CH₂CH₂O)ₓ(CH(CH₃)CH₂O)_{y}(CH₂CH₂O)ₓ H

Suitably, the mean value of x in Formula V is 4 or more, preferably 8 or more, more preferably 25 or more, yet more preferably 50 or more and most preferably 80 or more. The mean value of x is typically no greater than 200. Suitably, the mean value of y is 25 or more, preferably 35 or more, more preferably 45 or more and most preferably 60 or more. The mean value of y is typically no greater than 100.

Another preferred form of the surface active block polymer is according to Formula VI and has the CTFA designation Poloxamine. Those are commercially available under the trade name "Tetronic" from BASF:

VI) (HO(CH₂CH₂O)ₐ(CH(CH₃)CH₂O)_{b})₂-N-CH₂-CH₂-N-((OCH₂CH(CH₃))_{b}(OCH₂CH₂)ₐ OH)₂

Suitably, the mean value of a is 2 or more, preferably 4 or more, more preferably 8 or more, even more preferably 25 or more and most preferably 40 or more. The mean value of a is typically no greater than 200. The mean value of b is suitably 6 or more, preferably 9 or more, more preferably 11 or more and most preferably 15 or more. The mean value of b is typically no greater than 50.

Preferably, the surface active block polymer is poloxamer and/or poloxamine, more preferably, the surface active block polymer is poloxamer.

Preferably, the surface active block polymer is blended with dimethicone. The weight ratio of dimethicone to surface active block polymer in the blend is preferably in the range from 2:1 to 200:1, more preferably from 5:1 to 50:1, even more preferably from 10:1 to 40:1, most preferably from 15:1 to 30:1.

The water-insoluble conditioning agent is generally present in the composition in an amount from 0.05 to 15%, preferably from 0.1 to 10%, more preferably from 0.5 to 8%, most preferably from 1 to 5%, based on the total weight of the composition and including all ranges subsumed therein.

The composition may preferably comprise a pearlescer. The preferred pearlescer is ethylene glycol ester as disclosed in US4885107, incorporated herein by reference. Preferably, the ethylene glycol ester is a mono- or di-ester of glycol, more preferably a di-ester of glycol.

Preferably ethylene glycol mono- or di-ester is an ethylene glycol mono- or di-ester of a C₁₂₋₂₂ fatty acid, more preferably an ethylene glycol mono- or di-ester of a saturated C₁₂₋₂₂ fatty acid. Most preferred are the diesters comprising a mixture of palmitate and stearate. The amount of stearate should be in the range of about 10% to about 42% or in the range of about 55% to about 80% with palmitate accounting for the remainder. The amount of stearate is preferably from about 60% to about 75%, more preferably from about 80-95%, most preferably 100%. The most suitable example of a pearlescer is an ethylene glycol distearate. Pearlescer may also perform the function as a suspending agent.

The level of the ethylene glycol ester can be suitably from about 0.5% to about 6%, preferably from about 1% to about 4%, by weight of the total composition.

The viscosity of the composition suitably ranges from 3,000 to 10,000 mPa.s, preferably from 4,000 to 8,000 mPa.s, more preferably from 5,000 to 7,000 mPa.s when measured using a Brookfield V2 viscometer (spindle RTV5, 1 minute, 20rpm) at 30°C.

Compositions of the composition may comprise an oil. A preferred oil for use with the invention is coconut oil.

Coconut oil typically may have the following fatty acid content:

| **Type of Fatty Acid** | **%** |
|---|---|
| Caprylic (saturated, C₈) | 7 |
| Decanoic (saturated, C₁₀) | 8 |
| Lauric (saturated, C₁₂) | 48 |
| Myristic (saturated, C₁₄) | 16 |
| Palmitic (saturated, C₁₆) | 9.5 |
| Oleic (monounsaturated, C_{18:1}) | 6.5 |
| Other | 5 |

Preferably, the coconut oil may be present in any topical composition at a level of 0.1 to 10 wt%, preferably 0.5 to 5 wt%, more preferably 1.0 to 4 wt% of the topical cosmetic composition.

The pH of the composition of the invention preferably ranges from 3 to 9, more preferably from 5 to 7, still more preferably from 5.5 to 6.5.

The present invention is illustrated by means of the following non-limiting examples.

### EXAMPLE 1

To demonstrate the rapid action of piroctone olamine compared with zinc pyrithione the following Examples were prepared:
Example 1 - 3.1 wt% piroctone olamine (Octopirox OCT) in coconut oil;
Example A - coconut oil
Example B - 3.1 wt% of zinc pyrithione (ZnPTO) in coconut oil

The rapid effectiveness of Example 1 was tested by anti-Malassezia efficacy of actives solubilized in oil.

Pityrosporum Broth (PB) agar plates were prepared. *M. furfur* (CBS 1878) was initially grown in PB and adjusted to a final concentration of 10⁶ cells/ml in fresh PB medium before use. Aliquots of 200 µl of 10⁶ cfu/ml *M. furfur* was spread onto the agar surface using a spreading rod. Three, 2mm diameter plugs of agar were aseptically removed from inoculated plates and discarded, to create 'wells'.

**Pityrosporum Broth (PB)**

| | |
|---|---|
| 10g | Bacteriological Peptone |
| 0.1g | Yeast extract |
| 10g | Ox-bile |
| 2.5g | Taurocholic acid |
| 10g | Glucose |
| 1L | Deionised water |
| 0.5ml | Tween60 |
| 1ml | Glycerol |
| Adjust | pH to 6.2 |

| After sterilization | |
|---|---|
| 0.5ml | UHT milk |

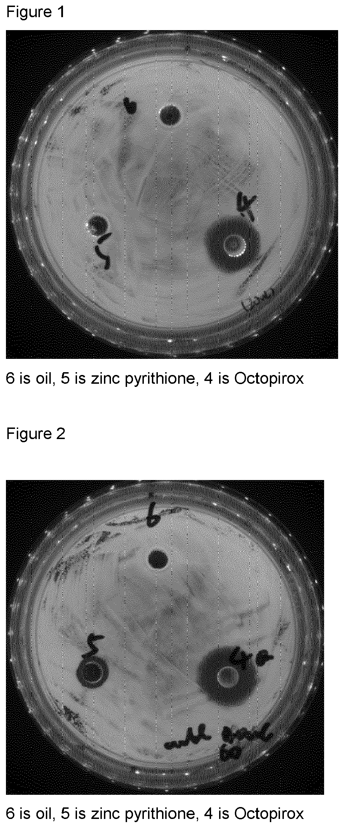

Three test materials were prepared as follows: Example 1, where 3.1wt% piroctone olamine mixed in coconut oil (labelled sample 4 in Figures 1 & 2); Example A, where coconut oil was used (labelled sample 6 in Figures 1 & 2); Example B, where 3.1 wt% zinc pyrithione mixed in coconut oil (labelled sample 5 in figures 1 & 2). All test materials were left overnight, then filtered using 0.22 µm pore size syringe filter. Resulting filtrates were added to the wells. Agar plated were incubated (32°C) for 3 days.
Figure 1 is a photograph of an agar plate in which 20 µl have each of the above Examples have been added.
Figure 2 is a photograph of an agar plate in which 60 µl have each of the above Examples have been added.

The Examples demonstrate that for the incubation time Octopirox is more effective in creating an exclusion zone than zinc pyrithione.

### EXAMPLE 2

The following is an example of a shampoo composition comprising piroctone olamine.

| Ingredient (INCI name) | wt% |
|---|---|
| Sodium Laureth Sulphate | 16-20 |
| Cocamidopropyl Betaine | 4-6 |
| Piroctone Olamine | 0.3-1.0 |
| Guar Hydroxypropyltrimmonium chloride | 0.2 |
| Carbo mer | 0.3 |
| Ethylene Glycol Distearate | 3.0 |
| Silicone Oil | 3.0 |
| Aqua + Minors (fragrance, pH and viscosity adjustors) | To 100% |

The composition in the table above may be suitably used in the cosmetic use according to the present invention.

## Claims

1. Cosmetic use of piroctone olamine to decrease the level of genus Malassezia species on a surface.

2. Use according to claim 1 in which the surface is skin.

3. Use according to any of the preceding claims, wherein the surface is scalp, preferably human scalp.

4. Use according to any of the preceding claims, wherein piroctone olamine is applied from a cosmetic composition, preferably a shampoo.

5. Use according to claim 4, wherein the level of piroctone olamine in the composition is from 0.01 to 5.0 %, by weight of the total composition.

6. Use according to claim 4 or claim 5 in which the composition is a shampoo composition.

7. Use according to any one of claims 4 to 6, wherein the composition comprises an anionic cleansing surfactant at a level of from 1 to 45%, by weight of the total composition, preferably from 10 to 25 wt%.

8. Use according to any of claims 4 to 7, wherein the composition comprises a cationic deposition polymer, preferably a guar hydroxypropyltrimmonium chloride; the level of said polymer is from 0.01 to 5%, by weight of the total composition, preferably from 0.05 to 2 wt%, more preferably from 0.1 to 1 wt%.

9. Use according to any of claims 4 to 8, wherein the composition further comprises an oil.

10. Use according to claim 9, wherein the oil is coconut oil.

11. Cosmetic use of a combination of piroctone olamine and coconut oil in a shampoo to decrease the level of genus Malassezia species on the human scalp.

12. Use according to any of the preceding claims wherein the decrease is rapid.
